# EUROPÄISCHE PATENTSCHRIFT

(11) **EP 1 375 491 B1**
(45) Veröffentlichungstag und Bekanntmachung des Hinweises auf die Patenterteilung: **25.10.2006**
(21) Anmeldenummer: 03013771.5
(22) Anmeldetag: 18.06.2003
(51) Int. Cl.: C07D 315/00, C07D 311/94

(54) **Verfahren zur Herstellung von 11(12)-Pentadecen-15-oliden**
Process for the preparation of 11(12)-pentadecen-15-olides
Procédé de préparation de 11(12)-pentadécen-15-olides

(30) Priorität: 19.06.2002 DE 10227483
(43) Veröffentlichungstag der Anmeldung: 02.01.2004
(60) Teilanmeldung: 06114976.1
(73) Patentinhaber: Symrise GmbH & Co. KG, 37603 Holzminden (DE)
(72) Erfinder: Esser, Peter, Summerville, SC 29485 (US); Koch, Oskar, 37079 Göttingen (DE); Marks, Werner, 37647 Brevörde (DE); Klein, Jared, Summerville, SC 29485 (US)
(74) Vertreter: Eisenführ, Speiser & Partner

(56) Entgegenhaltungen:
- EP-A- 0 503 312
- EP-A- 0 862 911
- WO-A-97/32948
- FR-A- 2 043 784
- YU N. OGIBIN ET AL.: "RING EXPANSION REACTION OF 1-HYDROPEROXY-16-OXABICYCLO(10.4.0)HEXADEC ANE CATALYSED BY COPPER IONS:" RUSSIAN CHEMICAL BULLETIN., Bd. 47, Nr. 6, Juni 1998 (1998-06), Seiten 1166-9, XP002254763 PLENUM PUBLISHING CO, NEW YORK, NY., US ISSN: 1066-5285

## Beschreibung

Die Erfindung betrifft ein verbessertes Herstellungsverfahren für 1l(12)-Pentadecen-15-olide.

Die makrocyclischen Lactone 11-Pentadecen-15-olid (=15-Hydroxypentadec-11-ensäurelacton) und 12-Pentadecen-15-olid (=15-Hydroxypentadec-12-ensäurelacton) sowie deren Gemische (11(12)-Pentadecen-15-olide) sind bekannte Moschusriechstoffe. Dabei sind sowohl die jeweiligen (E)- als auch (Z)-Formen als auch deren Gemische geruchlich interessant. In EP-A 424 787 sind die geruchlichen Eigenschaften dieser Substanzen beschrieben. Es ist ebenfalls hinreichend bekannt, dass aus den 11(12)-Pentadecen-15-oliden mittels Hydrierung 15-Pentadecanolid (15-Hydroxypentadecansäurelacton) erhalten werden kann, welches ebenfalls als Moschusriechstoff verwendet wird.

Die Herstellung von 11(12)-Pentadecen-15-oliden ist an sich bekannt. Die heute wichtigsten Herstellungsverfahren gehen von 13-Oxabicyclo[10.4.0]hexadec-1(12)-en (DDP) aus. Mittels säurekatalysierter Addition von Wasserstoffperoxid an DDP wird 1-Hydroperoxy-16-oxabicyclo[10.4.0]hexadecan (DDP-OOH) erhalten. Als entscheidender Schritt in der Synthese zu den 11(12)-Pentadecen-15-oliden ist die Spaltung des DDP-OOH unter Bildung des makrocyclischen Rings zu sehen. Diese Spaltung wird meist in Gegenwart von Katalysatoren wie Cu(OAc)₂ und gegebenenfalls FeSO₄ durchgeführt. Wird diese Reaktionsstufe rein thermisch durchgeführt, so enthält das Reaktionsprodukt erhebliche Mengen der gesättigten Verbindung 15-Pentadecanolid, welche zwar ein Moschusriechstoff ist, jedoch andere geruchliche Eigenschaften als die 11(12)-Pentadecen-15-olide aufweist und daher nur in möglichst geringen Mengen entstehen sollte. Darüber hinaus ist bei der rein thermischen Spaltung eine hohe Rückstandsbildung (z.B. Destillationssumpf) nachteilig.

DDP wird üblicherweise durch säurekatalysierte Cyclisierung von 2-(3-Hydroxypropyl)-1-cyclododecanon (OCP) unter Wasserabspaltung erhalten, welches wiederum durch radikalische Addition von Allylalkohol an Cyclododecanon synthetisiert werden kann (z.B. in DE-OS 2 136 496).

Der Prozess zur Herstellung der 11(12)-Pentadecen-15-olide kann durch das folgende Schema verdeutlicht werden:

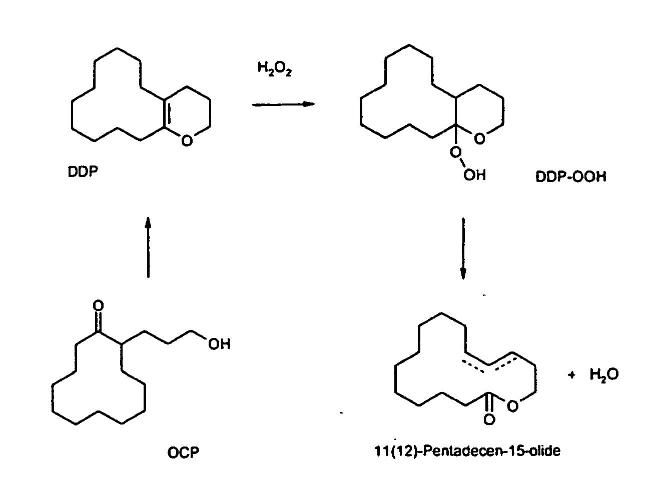

In EP-A 424 787 wurde OCP in 4,6 Gewichtequivalenten Eisessig bei Umgebungstemperatur homogenisiert, eine kalte 25 %ige wässrige Lösung von Schwefelsäure (etwa 51 mol% (etwa 21 Gew.%) bezogen auf OCP) zugegeben und die Reaktionsmischung anschließend auf 0°C abgekühlt. Danach wurden 1,65 molare Equivalente an Wasserstoffperoxid (70%ige Lösung) zugegeben, wobei die Temperatur auf 7°C anstieg. Nach einer kurzen Nachreaktionszeit wurde der gebildete Feststoff (DDP-OOH) abfiltriert, dieser mit Wasser und wässriger NaHCO₃-Lösung gewaschen und getrocknet; die Ausbeute betrug 80 %.

Die Spaltung des DDP-OOH erfolgte derart, dass das DDP-OOH in eine gesättigte Lösung von Cu(OAc)₂ in Methanol (hergestellt aus etwa 94 mol% Cu(OAc)₂ und 12,3 Gewichtsteilen Methanol bezogen auf das DDP-OOH; die Konzentration an DDP-OOH in dieser Methanol-Menge lag bei etwa 0,25 mol/l) portionsweise eingebracht wurde. Es folgte die Zugabe von 2 Portionen FeSO₄ (je knapp 20 mol% bezogen auf DDP-OOH) und Rühren bei Umgebungstemperatur über Nacht. Zur Aufarbeitung wurde auf gesättigte wässrige NaCl-Lösung gegeben, mit Diisopropylether extrahiert und dieser Extrakt mit gesättigter wässriger NaHCO₃-Lösung und gesättigter wässriger NaCl-Lösung gewaschen. Nach Trocknung und fraktionierter Destillation wurden 73 % der Theorie an 11(12)-Pentadecen-15-oliden erhalten, die noch 8 % 15-Pentadecanolid enthielten.

In Russ. Chem. Bull. 1998, 47, 1166-1169 wurde DDP in 5,2 Gewichtequivalenten Eisessig bei 0°C vorgelegt und eine Mischung bestehend aus einer 50 %igen wässrigen Lösung von Schwefelsäure (etwa 26 mol% (= 11 Gew.%) bezogen auf DDP) und 30 %igem Wasserstoffperoxid (etwa 1,89 molare Equivalente) zugegeben. Nach einer kurzen Nachreaktionszeit wurde der gebildete Feststoff (DDP-OOH) abfiltriert, dieser mit einer 50 %igen Essigsäurelösung (80 Gew.% bezogen auf DDP) und anschließend mehrfach mit Wasser (4 Waschvorgänge mit je 2 Gewichtsteilen Wasser bezogen auf DDP) bis zur Neutralität des Waschwassers gewaschen. Nach Trocknung des Feststoffes wurden 85 % der Theorie an DDP-OOH erhalten, das eine 96 %ige Reinheit aufwies.

Die Spaltung des DDP-OOH erfolgte derart, dass eine Suspension aus 1 Anteil DDP-OOH und etwa 3,8 Gewichtsanteilen 4-Methylpentan-2-on (MIBK) über einen längeren Zeitraum in eine siedende Lösung von Cu(OAc)₂ in etwa 3,8 Gewichtsanteilen MIBK (bezogen auf DDP-OOH) eindosiert wurde. Die Menge an Cu(OAc)₂ wurde im Bereich von 0,15 bis 7,0 mol%, bezogen auf das DDP-OOH, variiert, wobei bei 5 mol% Cu(OAc)₂ nach Angabe der Autoren das Optimum lag. Nach 3 Stunden der Nachreaktionszeit bei Siedehitze wurde das Reaktionsgemisch abgekühlt und von den ausgefallenen Kupfersalzen befreit. Das Filtrat wurde mit heißem Wasser (2 Waschvorgänge mit je 7,7 Gewichtsequivalenten Wasser bezogen auf DDP-OOH) gewaschen und eingeengt. Es wurde unter Verwendung von 5 mol% Cu(OAc)₂ eine Rohausbeute an 11(12)-Pentadecen-15-oliden von 96,5 % der Theorie erhalten.

Nachteile dieser Verfahren sind unter den Reaktionsbedingungen der Spaltung von DDP-OOH insbesondere das Ausfallen elementaren Kupfers und/oder unlöslicher Kupferverbindungen sowie die großen Mengen der in den Reaktionen verwendeten Reagenzien und Hilfsstoffe. Als weitere Nachteile sind beispielsweise die vielen, teils aufwendigen, Prozessschritte und Waschvorgänge zu nennen und die damit zusammenhängenden Umwelt-und Sicherheitsaspekte sowie schlechte Raum-Zeit-Ausbeuten. Bei einer Maßstabsvergrößerung des letztgenannten Verfahrens ist zudem nachteilig, dass erhebliche Mengen ringverengter Lactone mit einer ω-Hydroxyalkyl-Seitenkette gebildet werden.

Die bekannten Syntheseverfahren sind daher für eine industrielle Umsetzung ungeeignet. Ein technisches Verfahren, das auf einfache und kostengünstige Weise 11(12)-Pentadecen-15-olide liefert, ist deshalb von großem wirtschaftlichem Interesse.

Mit der vorliegenden Erfindung gelingt es, die genannten Nachteile zu überwinden, und ein technisch günstiges Verfahren bereit zu stellen. Das erfindungsgemäße Verfahren ist besonders für den Einsatz im industriellen Maßstab geeignet.

Gegenstand der vorliegenden Erfindung ist daher ein Verfahren zur Herstellung von 11(12)-Pentadecen-15-oliden ausgehend von DDP-OOH in Gegenwart einer Cu-(II)-verbindung und eines Verdünnungsmittels, dadurch gekennzeichnet, dass während der Reaktion ein Azeotrop enthaltend Wasser und Verdünnungsmittel abdestilliert wird, mit den weiteren Schritten gemäß Anspruch 1.

Erfindungsgemäß vorteilhafte Cu-(II)-verbindungen sind solche, die unter den Reaktionsbedingungen der DDP-OOH-Umsetzung in dem verwendeten Verdünnungsmittel löslich sind. Solche Cu-(II)-verbindungen weisen bei 20°C in dem Verdünnungsmittel eine Löslichkeit von mindestens 0,5 g/kg Verdünnungsmittel auf, bevorzugt von mindestens 1 g/kg. Die Cu-(II)-Verbindungen können wasserfrei oder als Hydrate (Kristallwasser) eingesetzt werden. Die Wassermenge des Kristallwassers ist unkritisch.

Bevorzugte Cu-(II)-verbindungen sind solche mit organischen Resten. Neben Cu-(II)-2,4-pentandionat-Derivaten sind dabei Cu-(II)-carboxylate besonders geeignet. Bevorzugte Cu-(II)-2,4-pentandionate sind Cu-(II-acetylacetonat, Cu-(II)-1,1,1-trifluoracetylacetonat und [Bis(2,2,6,6-tetramethyl-3,5-heptandionato)]-Cu-(II). Besonders bevorzugt sind Cu-(II)-carboxylate von Alkylcarbonsäuren mit 2 bis 5 Kohlenstoffatomen, insbesondere Cu-(II)-acetat und Cu-(II)-propionat.

Erfindungsgemäß können ein oder mehrere Cu-(II)-verbindungen verwendet werden. Die erfindungsgemäß vorteilhafte Menge der Cu-(II)-verbindungen bei der Umsetzung von DDP-OOH zu den 11(12)-Pentadecen-15-oliden beträgt 0,02 bis 1,5 mol% bezogen auf DDP-OOH, bevorzugt 0,05 bis 1 mol% und besonders bevorzugt 0,1 bis 0,6 mol%.

Als Verdünnungsmittel können alle flüssigen und mit Wasser Azeotrope bildenden organischen Verbindungen eingesetzt werden. Erfindungsgemäß füngieren diese Verdünnungsmittel bei der destillativen Entfernung des Verdünnungsmittels als Wasserschlepper. Es können ein oder mehrere Verdünnungsmittel verwendet werden, bevorzugt ist die Verwendung nur eines Verdünnungsmittels. Vorteilhafte Verdünnungsmittel haben bei Normaldruck einen Siedepunkt im Bereich 70 bis 230°C, besonders vorteilhaft im Bereich von 90 bis 150°C. Bevorzugte Verdünnungsmittel sind aliphatische Ester, Ether oder Ketone, insbesondere Ketone. Bevorzugter Ester ist Butylacetat, bevorzugter Ether ist Di-n-butylether Bevorzugte Ketone sind 2-Pentanon, 3-Pentanon, 4-Methylpentan-2-on (MIBK), Ethylisopropylketon und Diisopropylketon, besonders bevorzugt ist MIBK.

Es ist bevorzugt das bei der Spaltung abdestillierte Azeotrop vom Wasser zu befreien und das Verdünnungsmittel wieder für die Spaltung zu verwenden. Es ist dabei vorteilhaft, wenn dieses wiedergewonnene Verdünnungsmittel eine Säurezahl von kleiner oder gleich 3 mg KOH g aufweist. Die Säurezahl entspricht der Anzahl der Milligramm an Kaliumhydroxid, die zur Neutralisation eines Gramms des wiedergewonnenen Verdünnungsmittels benötigt werden.

Das erfindungsgemäß bevorzugte Gewichtsverhältnis von DDP-OOH zu der Gesamtmenge des in der Spaltung verwendeten Verdünnungsmittels liegt im Bereich 1 : 2 bis 1 : 7, bevorzugt bei 1 : 3 bis 1 : 5.

Der Temperaturbereich, in dem die Spaltung durchgeführt wird, liegt bei 70 bis 120°C. Bevorzugt wird die Spaltung bei Temperaturen im Bereich von 80 bis 100°C, besonders bevorzugt bei 85 bis 95°C durchgeführt.

Der vorteilhafte Druckbereich, in dem die Spaltung durchgeführt wird, liegt bei 0,01 mbar bis 2 bar. Bevorzugt wird das Verfahren bei Drücken unterhalb von 1013 mbar ausgeführt, insbesondere im Bereich von 50 bis 800 mbar. Wenn die Umsetzung diskontinuierlich erfolgt, so wird der Druck bevorzugt mit fortschreitender Spaltung immer weiter abgesenkt und die Temperatur der Spaltung weitgehend konstant gehalten, bevorzugt beginnend bei 450 bis 750 mbar und endend bei 100 bis 400 mbar.

Das in die Spaltung eingesetzte DDP-OOH kann wasserfrei oder wasserhaltig sein, bevorzugtes DDP-OOH ist wasserhaltig. Der Wassergehalt des DDP-OOH liegt vorteilhafterweise im Bereich von 5 bis 40 Gew.%, bevorzugt bei 10 bis 30 Gew.% und besonders bevorzugt bei 15 bis 25 Gew.%.

Überraschenderweise wurde zudem gefunden, dass bestimmte Additive ein Ausfallen von Kupfer und/oder unlöslichen Kupferverbindungen während der Spaltung unterdrücken. Erfindungsgemäß wird die Umsetzung von DDP-OOH zu den 11(12)-Pentadecen-15-oliden in Gegenwart von Additiven durchgeführt, die durch folgende Formel wiedergegeben werden können:

HX-[A]-YH,

wobei
- X und Y: unabhängig voneinander O oder N-R bedeuten, wobei R = H oder ein organischer Rest mit 1 bis 10 Kohlenstoffatomen ist, und
- A: ein organischer Rest umfassend 2 bis 100 Kohlenstoffatome ist.

Vorteilhafterweise liegt der Siedepunkt des Additivs oberhalb des Siedepunktes des verwendeten Verdünnungsmittels und oberhalb des azeotropen Gemisches von Verdünnungsmittel und Wasser.

Bevorzugt umfasst A 6 bis 50 Kohlenstoffatome, besonders bevorzugt 10 bis 30 Kohlenstoffatome. Der Rest R umfasst bevorzugt 1 bis 4 Kohlenstoffatome, bevorzugt ist R = Methyl oder Ethyl.

Der organische Rest A enthält bevorzugt die Heteroatome O oder N, bevorzugt in Form von Hydroxygruppen, Ethergruppen oder Aminogruppen, bevorzugt sind Ethergruppen und sekundäre Aminogruppen. An dem Kohlenstoffgerüst des Restes A können ein oder mehrere organische Gruppierungen angebracht sein, die unabhängig voneinander geradkettig, verzweigt, cyclisch, heterocyclisch, aromatisch oder heteroaromatisch sein können, wobei bei den heteroatomhaltigen Gruppierungen solche mit O oder N bevorzugt sind.

Vorteilhafte Additive sind α,ω-Diole und α,ω-Aminoalkohole.

In einer besonders vorteilhaften Ausführungsform werden Additive eingesetzt, die als Heteroatome ausschließlich Sauerstoff enthalten. Diese α,ω-Diole enthalten im Kohlenstoffgerüst des organischen Restes A bevorzugt mindestens 2 Sauerstoffatome, bevorzugt in Form von Ethergruppen.

Besonders bevorzugte Additive sind Polyalkylenglykole, insbesondere Polyethylenglykole (PEG), Polypropylenglykole oder Polytetramethylenglykole (Polytetrahydrofurane), welche mindestens einen Polymerisationsgrad von 2 und vorzugsweise Molmassen im Bereich von 144 bis etwa 2200 aufweisen. Die Polyalkylenglykole sind bei höheren Molmassen polydispers und haben einen Molmassenbereich, z.B. weist PEG 1000 typischerweise einen Molmassenbereich von 950 bis 1050 auf. Ganz besonders bevorzugt sind Polyethylenglykole (PEG) mit Polymerisationsgraden von 4 bis 50. In besonderem Maße bevorzugt sind PEG 200 bis PEG 1000, hierbei wiederum PEG 400, PEG 600 und PEG 800. Diese Produkte sind handelsüblich verfügbar.

Erfindungsgemäß können ein oder mehrere Additive verwendet werden. Die erfindungsgemäß vorteilhafte Menge der Additive bei der Umsetzung von DDP-OOH zu den 11(12)-Pentadecen-15-oliden beträgt 0,5 bis 15 Gew.% bezogen auf DDP-OOH, bevorzugt 1 bis 10 Gew.%, besonders bevorzugt 2 bis 8 Gew.% und ganz besonders bevorzugt 3 bis 6 Gew.%.

Die Spaltung wird bevorzugt dergestalt durchgeführt, dass eine Mischung enthaltend Verdünnungsmittel und Cu-(II)-verbindung sowie Additiv vorgelegt und aufgeheizt wird. Zu dieser geheizten Mischung wird dann eine Suspension oder Lösung enthaltend Verdünnungsmittel und DDP-OOH zugegeben. Es ist besonders vorteilhaft, die azeotrope destillative Entfernung des wasserhaltigen Verdünnungsmittels mit Dosierbeginn des DDP-OOH zu starten.

Nach beendeter Spaltung ist es vorteilhaft, die Kupferionen vor einer destillativen Isolierung der 11(12)-Pentadecen-15-olide weitestgehend zu deaktivieren. Hierzu ist es zweckdienlich, das Kupfer stark zu komplexieren und/oder als Kupferverbindung auszufüllen. Hierzu sind insbesondere starke Komplexbildner des Kupfers geeignet, wie z.B. Kryptanden, EDTA oder Citronensäure.

Die Spaltung kann diskontinuierlich, semi-kontinuierlich oder kontinuierlich durchgeführt werden, bevorzugt ist die semi-kontinuierliche oder kontinuierliche Verfahrens führung.

Die Spaltung kann auch in einen kontinuierlich betriebenen Rührkesselreaktor ausgeführt werden. Es wird dann bevorzugt nicht nur eine Suspension aus DDP-OOH und Verdünnungsmittel dosiert, sondern parallel eine Suspension einer Cu-(II)-verbindung, Verdünnungsmittel sowie gegebenenfalls eines Additiv-Zusatzes. Man lässt die aus dem Rührkesselreaktor abgenommene rohe Suspension vorteilhafierweise noch durch einen zweiten kontinuierlich betriebenen Rührkesselreaktor, in dem weiteres wasserhaltiges Verdünnungsmittel abdestilliert wird, zur Nachreaktion laufen. Nach Bedarf kann in einem dritten kontinuierlichen betriebenen Rührkesselreaktor ein starker Cu-Komplexbildner eindosiert und die Destillation des wasserhaltigen Verdünnungsmittels fortgesetzt werden. Der Ablauf dieses Rührkesselreaktors kann auf eine kontinuierlich betriebene Destillationskolonne geleitet, bei der über Kopf Verdünnungsmittel und Reste an Wasser abgenommen werden und als Sumpfprodukt ein Verdünnungsmittel-freies Rohprodukt erhalten wird. Wurde ein starker Cu-Komplexbildner zugesetzt, so kann der gegebenenfalls ausgefallene Cu-Komplex abfiltriert werden und die rohen 11(12)-Pentadecen-15-olide weiter nach Bedarf gereinigt werden.

Typischerweise werden die 11(12)-Pentadecen-15-olide, sowohl bei kontinuierlich, semi-kontinuierlich oder diskontinuierlich durchgeführtem Verfahren, in einer isolierten Ausbeute von 97 % d.Th. bei der Spaltung und von 87 % d.Th. über alle Schritte ausgehend von DDP erhalten.

Das in erfindungsgemäßen Verfahren eingesetzte DDP-OOH wird vorzugsweise ausgehend von DDP und Wasserstoffperoxid in Gegenwart von Essigsäure und Schwefelsäure hergestellt, wobei die Umsetzung in Gegenwart von 0,05 bis 5 mol% H₂SO₄ bezogen auf DDP durchgeführt wird.

Die erfindungsgemäß bevorzugte Menge an H₂SO₄ bei der Umsetzung von DDP zu DDP-OOH beträgt 0,1 bis 3 mol% bezogen auf DDP und besonders bevorzugt 0,2 bis 2,5 mol%.

Das erfindungsgemäß bevorzugte Gewichtsverhältnis von DDP zu Essigsäure liegt im Bereich 1 : 2 bis 1 : 8, bevorzugt bei 1 : 4 bis 1 : 6.

Für einen spontanen und gleichmäßigen Reaktionsverlauf ist es vorteilhaft, einen Teil des vorherigen Rohansatzes (nicht filtrierte rohe Reaktionsproduktmischung, gegebenenfalls noch DDP-OOH Kristalle enthaltend, deren Hauptbestandteil Essigsäure ist) vorzulegen.

Für die Addition von H₂O₂ an DDP zu DDP-OOH kann Wasserstoffperoxid unterschiedlichen Gehalts verwendet werden. Typischerweise wird 30 bis 70 %iges wässriges Wasserstoffperoxid eingesetzt, bevorzugt ist 50 oder 70%iges.

Die erfindungsgemäß bevorzugte Menge an H₂O₂ beträgt 0,8 bis 1,25 molare Equivalente bezogen auf DDP und besonders bevorzugt 0,95 bis 1,1 molare Equivalente.

Der Temperaturbereich, in dem die Addition von H₂O₂ durchgeführt wird, liegt bei 0 bis 10°C, bevorzugt bei 3 bis 8°C. Als im besonderen Maße geeignet erweist sich eine Reaktionstemperatur um 5°C.

Als Ausgangsmaterial für die Herstellung von DDP-OOH kann auch OCP verwendet werden. Hierbei ist es dann vorteilhaft mehr Essigsäure einzusetzen, typischerweise 10 bis 30 Gew.% mehr als bei der Verwendung von DDP als Edukt.

Nach beendeter Reaktion wird das Reaktionsgemisch filtriert und die DDP-OOH-Kristalle mit möglichst wenig Wasser gewaschen, um die Kristalle weitgehend von Essigsäure und Schwefelsäure zu befreien. Vorteilhaft ist eine zweifache Gegenstromwäsche mit Wasser, jeweils mit der doppelten Gewichtsmenge an Wasser bezogen auf DDP-OOH. Für diese Filtration eignen sich insbesondere Bandfilter und Stülpzentrifugen. Für die Waschvorgänge werden bevorzugt Wasser (z.B. demineralisert oder destilliert) und/oder Waschwässer vorheriger DDP-OOH-Kristallwäschen verwendet. Es ist besonders vorteilhaft, die nach Filtration und den Waschvorgängen vorliegenden DDP-OOH-Kristalle nicht weiter zu trocknen, sondern den erhaltenen Wassergehalt beizubehalten.

Das nach der Filtration der DDP-OOH-Kristalle und den sich anschließenden Waschvorgängen erhaltene Filtrat (kristallfreie Reaktionslösung) enthält hauptsächlich Essigsäure. Der Wassergehalt in diesem Filtrat beträgt meist 10 bis 25 Gew.%. Dieses Filtrat wird bevorzugt erneut für dieselbe Reaktion, zumeist nach Zugabe eines Anteils frischen Eisessigs, eingesetzt.

Die Waschvorgänge und die Filtration und die gegebenenfalls erfolgende Trocknung der gewaschenen DDP-OOH Kristalle sollten möglichst schonend und bei Temperaturen im Bereich von -10 bis +10°C erfolgen, bevorzugt ist eine Temperatur um 0°C.

Die nach Filtration, Waschvorgängen und gegebenenfalls Trocknung vorliegenden DDP-OOH-Kristalle weisen bevorzugt einen Wassergehalt von 5 bis 40 Gew.%, besonders bevorzugt von 10 bis 30 Gew.% und ganz besonders bevorzugt von 15 bis 25 Gew.% auf.

Die Herstellung von DDP-OOH kann diskontinuierlich, semi-kontinuierlich oder kontinuierlich durchgeführt werden.

Die Reaktion kann beispielsweise in einen kontinuierlich betriebenen Rührkesselreaktor ausgeführt werden. Es wird dann Wasserstoffperoxid einerseits und parallel eine Mischung enthaltend DDP, Essigsäure, Schwefelsäure und Wasser andererseits in diesen Rührkesselreaktor dosiert. Diese Mischung kann auch durch DDP, kristallfreie Reaktionslösung (z.B. aus dem Voransatz), etwas frische Schwefelsäure und frische Essigsäure und gegebenenfalls Wasser hergestellt werden. Lässt man die aus dem Rührkesselreaktor abgenommene Suspension noch durch einen zweiten kontinuierlich betriebenen Rührkesselreaktor zur Nachreaktion laufen, so unterscheidet sich die kontinuierliche Fahrweise nicht von der diskontinuierlichen (Batch) Variante in Bezug auf Ausbeute und Qualität des Produktes.

Typischerweise wird DDP-OOH, sowohl bei kontinuierlich, semi-kontinuierlich oder diskontinuierlich durchgeführtem Verfahren, in einer isolierten Ausbeute von 90 % d.Th. ausgehend von DDP erhalten.

Es ist empfehlenswert alle Schritte bei der Herstellung der 11(12)-Pentadecen-15-olide, insbesondere die Herstellung, Filtration und Handhabung der DDP-OOH-Kristalle, in einer Atmosphäre von Schutzgas oder Mischungen von Schutzgasen durchzuführen. Besonders geeignete Schutzgase sind beispielsweise Stickstoff, Argon, Helium oder Kohlendioxid.

Dass die Reaktion von DDP mit Wasserstoffperoxid zum DDP-OOH kontinuierlich, die Trennung von Reaktionslösung und DDP-OOH-Kristallen sowie deren Reinigung quasi kontinuierlich und die Umlagerung von DDP-OOH unter Kupfer-(II)-Katalyse zu den 11(12)-Pentadecen-15-oliden ebenfalls kontinuierlich ausgeführt werden kann, ist nicht nur unter ökonomischen Aspekten wichtig. Unter dem Aspekt von Sicherheit für Arbeit und Umwelt kann so die im Prozess befindliche Menge an Hydroperoxiden auf ein Miniumum verringert werden.

Mit dem vorliegenden Verfahren gelingt es, die Lösungsmittel- und Katalysator-Kreisläufe zu schließen bzw. die Abfälle auf ein Minimum zu reduzieren.

Die folgenden Beispiele erläutern die Erfindung.

### Beispiel 1

### Herstellung von DDP-OOH

In ein 6 L Doppelmantelgefäß mit Bodenablauf, versehen mit Blattrührer, 2 Dosierpumpen, Schlauchpumpe, Kryostat und Stickstoffanschluss wurden 448 g DDP, 2070 g Essigsäure, 330 g entmineralisiertes Wasser und 4 g Schwefelsäure auf 5°C thermostatisiert vorgelegt. Es wurde mit 400 UpM (Umdrehungen pro Minute) gerührt. Innerhalb von 30 Minuten wurden 140 g 50 %iges Wasserstoffperoxid dosiert. Die Reaktion war exotherm und spontan. Nach 15 Minuten Nachreaktionszeit wurde die Reaktionsmischung über eine Porzellan-Nutsche filtriert und zweimal mit je 1000 g Wasser gewaschen (die vereinigten Filtrate bilden die kristallfreie Reaktionslösung). Die Ausbeute an DDP-OOH (ohne Wassergehalt von etwa 17 Gew.%) lag bei 89 % der Theorie.

### Beispiel 2

### Herstellung von DDP-OOH

In ein 6 L Doppelmantelgefäß mit Bodenablauf, versehen mit Blattrührer, Dosierpumpe, Kryostat und Stickstoffanschluss wurden 2600 g kristallfreie Reaktionslösung aus Voransatz (aus Beispiel 1), 448 g DDP, 180 g Essigsäure und 0,4 g Schwefelsäure auf 5°C thermostatisiert vorgelegt. Es wurde mit 400 UpM gerührt. Innerhalb von 30 Minuten wurden 100 g 70 %iges Wasserstoffperoxid dosiert. Die Reaktion war exotherm und spontan. Die Nachreaktionszeit betrug 15 Minuten. 300 g der rohen Reaktionsproduktmischung verblieben für den Folgeansatz im Reaktor. Die übrigen 3000 g wurden der weiteren Aufreinigung zugeführt.

### Beispiel 3

### Kontinuierliche Herstellung von DDP-OOH

In ein 6 L Doppelmantelgefäß mit Bodenablauf ausgestattet mit Blattrührer, 2 Dosierpumpen, Schlauchpumpe, Kryostat und Stickstoffanschluss wurden 300 g des vorherigen Rohansatzes (nicht filtrierte rohe Reaktionsproduktmischung, noch DDP-OOH Kristalle enthaltend), 448 g DDP, 2070 g Essigsäure, 330 g entmineralisiertes Wasser und 4 g Schwefelsäure auf 5°C thermostatisiert vorgelegt. Es wurde mit 400 UpM gerührt. Innerhalb von 30 Minuten wurden 140 g 50 %iges Wasserstoffperoxid eindosiert. Die Reaktion war exotherm und spontan. Nach 15 Minuten wurde die Reaktion kontinuierlich fortgesetzt, indem mit zwei Dosierpumpen 70 %iges Wasserstoffperoxid in der einen Pumpe und ein Dosiergemisch aus kristallfreier Reaktionslösung, DDP, Essigsäure und Schwefelsäure in der anderen Pumpe parallel in das Reaktionsgefäß eingebracht wurde. Gleichzeitig wurde mittels einer Schlauchpumpe über den Bodenablass Reaktionslösung abgenommen. Es wurde Wasserstoffperoxid 70 %ig mit einer Geschwindigkeit von 200 g/h dosiert. Das Dosiergemisch wurde in einem Verhältnis von 2300 g kristallfreier Reaktionslösung, 448 g DDP, 180 g Essigsäure und 0,4 g Schwefelsäure angesetzt und mit einer Geschwindigkeit von 5857 g/h eindosiert. Die Reaktionslösung wurde mit einer Geschwindigkeit von etwa 6057 g/h derart entnommen, dass das Flüssigkeitsniveau im Reaktor weitgehend konstant blieb. Der Rohansatz wurde der Filtration und der weiteren Aufreinigung zugeführt.

### Beispiel 4

### Filtration von DDP-OOH

Zur Trennung der DDP-OOH Kristalle von ihrer Mutterlauge wurde eine Stülpzentrifuge genutzt. Diese wurde vor Beginn des Schleuderprozesses auf etwa +5°C gekühlt und mit Stickstoff inertisiert. Die Zentrifuge wurde auf eine Schleuderdrehzahl von 2300 UpM eingestellt. 15 kg der Reaktionslösung wurden mit einer Schlauchpumpe innerhalb von 1 bis 1,5 Minuten eingepumpt. Die Dosierleitung wurde mit 2 kg kristallfreier Reaktionslösung nachgespült. Für 5 Minuten wurde mit 2300 UpM geschleudert. Es fielen 13,9 kg kristallfreier Reaktionslösung an, die bis zum Wiedereinsatz auf 1°C thermostatisiert wurden. Nach Reduzierung der Schleuderdrehzahl auf 600 UpM wurde die Zentrifuge durch Stülpen ausgeräumt. Man erhielt 3,1 kg feuchte Kristalle an DDP-OOH.

Die Kristalle wurden bei etwa 1°C mit 6,9 kg Waschwasser angemaischt und 15 Minuten kräftig gerührt. Als Waschwasser kann entmineralisiertes Wasser oder das Filtrat vorheriger Filtrationsansätze eingesetzt werden (Essigsäure-Gehalt etwa 3 Gew.%).

Die Stülpzentrifuge wurde auf eine Schleuderdrehzahl von 2600 UpM eingestellt. Mit einer Schlauchpumpe wurden 10 kg DDP-OOH-Suspension wurden innerhalb von 1 Minute eindosiert. Es wurde eine weitere 1 Minute mit unveränderter Drehzahl zwischengeschleudert. Es fielen 6,1 kg Abwasser an, das verworfen wurde. Bei gleichbleibender Drehzahl wurde mit 5,9 kg auf 1°C vorgekühltem entmineralisierten Wasser, das innerhalb von 1 Minute mit einer Dosierpumpe eingepumpt wurde, gewaschen. Es wurde danach 10 Minuten lang bei 2600 UpM geschleudert. Es fielen 6,9 kg Waschwasser an (die im nachfolgenden Ansatz wieder eingesetzt werden). Nach beenden des Schleuderns wurde die Schleuderdrehzahl auf 600 UpM reduziert. Die Zentrifuge wurde durch Stülpen ausgeräumt. Es wurden 2,9 kg DDP-OHH-Kristalle mit einem Feuchtegehalt von ca. 20 % erhalten, was einer Menge von 2,30 kg DDP-OOH entspricht. Die Ausbeute lag somit bei 90 % d.Th.. bezogen auf DDP umfassend die Stufen DDP-OOH-Bildung und dessen Isolierung. Das Produkt war praktisch frei von Essigsäure und Schwefelsäure.

### Beispiel 5

### Herstellung von 11(12)-Pentadecen-15-oliden

In ein mit Stickstoff inertisiertes 6 L Doppelmantelgefäß ausgestattet mit Rührer, Thermostat, Destillationskolonne, Kolonnenkopf, Vakuumpumpe und Vakuumregelung wurde eine Mischung aus 800 g MIBK 100 g PEG 400 und 3,6 g Kupfer-IIacetat-Monohydrat vorgelegt. Es wurde aufgeheizt und 30 Minuten lang unter Rückfluss gekocht (Kopftemperatur etwa 112°C, Sumpftemperatur etwa 118°C) . Es wurde vorsichtig ein Vakuum von 550 mbar eingeregelt, so dass sich eine Sumpftemperatur von 90°C und eine Kopftemperatur von 85°C einstellte.

2,9 kg des DDP-OOH (feucht, ca. 20 Gew.% Wasser, aus Beispiel 4) und 9 kg MIBK (gegebenenfalls aus der DDP-OOH-Spaltung wiedergewonnenes MIBK) wurden in einer auf 0°C thermostatisierten Dosiervorlage mit Rührer angemaischt. Aus der Dosiervorlage wurden von dieser Suspension etwa 2 L/h mittels einer Schlauchpumpe in das 6 L Doppelmantelgefäß eingebracht. Mit Beginn der Dosierung wurde am Kolonnenkopf etwas weniger an MIBK abgenommen als mit der Suspension in das Doppelmantelgefäß eindosiert wurde. Es wurde solange azeotrop abdestilliert, bis sich kein Wasser mehr abscheidet. So waren am Ende der Dosierung 8 kg MIBK (Säurezahl 2,0 mg KOH/g) und etwa 600 g Wasser abdestilliert. Das MIBK kann vorteilhafterweise wiederverwendet werden. Im Laufe der Dosierung wurde, um die Sumpftemperatur bei etwa 90°C zu halten, das Vakuum auf etwa 350 mbar abgesenkt. Dabei fiel die Kopftemperatur bis auf etwa 70°C.

Nach Abbruch der Destillation wurde die Heizung abgestellt und mit Stickstoff begast. Nach Zugabe von 5 g Citronensäure wurde unter Rühren bei Normaldruck aufgeheizt und solange destilliert, bis sich kein Wasser mehr abscheidet. Die Sumpftemperatur lag dann bei ca. 120°C und die Kopftemperatur bei etwa 115°C. Unter vorsichtigem Anlegen von Vakuum wurde die Destillation fortgesetzt. Bei einer Sumpftemperatur von 120°C und einem Vakuum von etwa 60 mbar wurde die Destillation abgebrochen und mit Stickstoff begast. Das erhaltene Destillat bestand aus etwa 30 g Wasser und 1,6 kg MIBK. Bei 50°C bis 60°C wurde das Rohprodukt über eine Porzellan-Nutsche filtriert und zweimal mit 100 g MIBK gewaschen. Das Filtrat und die beiden Waschfiltrate wurden getrennt aufgefangen. Es wurden 2250 g Filtrat und 6,2 g trockenes Kupfercitrat sowie ca. 250 g Waschfiltrate erhalten. Durch einengen können aus den Waschfiltraten weitere 50 g Filtrat gewonnen werden.

Nach erneuter Destillation wurden 40 g Vorläufe, 2080 g 11(12)-Pentadecen-15-olide und 150 g Destillationsrückstände, die unter anderem das PEG 400 enthalten, erhalten. Das entspricht einer Ausbeute an 11(12)-Pentadecen-15-oliden von 87 % d.Th. über alle Schritte ausgehend von DDP und einer Ausbeute von 97 % d.Th. für den Schritt vom DDP-OOH zu den 11(12)-Pentadecen-15-oliden.

### Beispiel 6

### Kontinuierliche Herstellung von 11(12)-Pentadecen-15-oliden

Es wird wie in Beispiel 5 verfahren, bis zum Ende der Dosierung und dem Punkt der azeotropen Destillation, an dem 8 kg MIBK und etwa 600 g Wasser abdestilliert worden waren.

An diesem Punkt wurde die Reaktion kontinuierlich fortgesetzt, indem weiterhin über die Schlauchpumpe eine DDP-OOH-Suspension, über eine zweite Dosierpumpe ein zweites Dosiergemisch eindosiert und über eine dritte Schlauchpumpe über den Bodenablass fortlaufend Reaktionslösung entnommen wurde. Die DDP-OOH-Suspension wurde in einem Verhältnis von 2,9 kg DDP-OOH (feucht, ca. 20 Gew.% Wasser, aus Beispiel 4) zu 9 kg MIBK angesetzt und mit einer Dosiergeschwindigkeit von 1,7 kg/h dosiert. Das zweite Dosiergemisch wurde aus 800 g MIBK, 100 g PEG 400 und 3,6 g Cu(OAc)₂-Monohydrat angesetzt und als sehr gut gerührte, auf 110°C vorgeheizte Suspension verwendet. Dieses zweite Dosiergemisch wurde mit einer Dosiergeschwindigkeit von 130 g/h dosiert. Über den Kolonnenkopf wurden 1230 g wasserhaltiges MIBK pro Stunde abdestilliert. Über den Bodenablass wurden 600 g/h Reaktionslösung abgeführt.

Die weitere Aufarbeitung der Reaktionslösung erfolgte entsprechend Beispiel 5 mit den gleichen Ergebnissen hinsichtlich der Ausbeuten.

## Patentansprüche

1. Verfahren zur Herstellung von 11(12)-Pentadecen-15-oliden ausgehend von 1-Hydroperoxy-16-oxabicyclo[10.4.0]hexadecan (DDP-OOH) in Gegenwart einer Cu-(II)-verbindung und eines Verdünnungsmittels, **dadurch gekennzeichnet, dass** während der Reaktion ein Azeotrop enthaltend Wasser und Verdünnungsmittel abdestilliert wird, wobei
die Reaktion in Gegenwart von Additiven der Formel
HX-[A]-YH,
durchgeführt wird, wobei
X und Y unabhängig voneinander O oder N-R bedeuten, wobei R = H oder ein organischer Rest mit 1 bis 10 Kohlenstoffatomen ist, und
A ein organischer Rest umfassend 2 bis 100 Kohlenstoffatome ist.

2. Verfahren nach Anspruch 1, **dadurch gekennzeichnet, dass** die Menge der Cu-(II)-verbindung 0,02 bis 1,5 mol%, bezogen aufDDP-OOH, beträgt.

3. Verfahren nach Anspruch 1 oder 2, **dadurch gekennzeichnet, dass** das Gewichtsverhältnis von DDP-OOH zu der Gesamtmenge des in der Spaltung verwendeten Verdünnungsmittels im Bereich 1 : 2 bis 1 : 7 liegt.

4. Verfahren nach mindestens einem der Ansprüche 1 bis 3, **dadurch gekennzeichnet, dass** zumindest ein Teil des Verdünnungsmittels aus vorherigen Ansätzen wiedergewonnen ist

5. Verfahren nach mindestens einem der Ansprüche 1 bis 4, **dadurch gekennzeichnet, dass** die Temperatur der Reaktion im Bereich von 80 bis 100°C liegt.

6. Verfahren nach mindestens einem der Ansprüche 1 bis 5, **dadurch gekennzeichnet, dass** die Additive Polyalkylenglykole sind.

7. Verfahren nach mindestens einem der Ansprüche 1 bis 6, **dadurch gekennzeichnet, dass** die Menge an Additiven bei 0,5 bis 15 Gew.% bezogen auf DDP-OOH liegt.

8. Verfahren nach mindestens einem der Ansprüche 1 bis 7, **dadurch gekennzeichnet, dass** der Wassergehalt des DDP-OOH vorteilhafterweise im Bereich von 5 bis 40 Gew.% liegt

9. Verfahren nach mindestens einem der Ansprüche 1 bis 8, **dadurch gekennzeichnet, dass** das Verfahren bei Drücken im Bereich von 50 bis 800 mbar durchgeführt wird.

10. Verfahren zur Herstellung von 11(12)-Pentadecen-15-oliden nach Anspruch 1; wobei in einem ersten Schritt 13-Oxabicyclo[10.4.0]hexadec-1(12)-en (DDP) und Wasserstoffperoxid in Gegenwart von Essigsäure und 0,05 bis 5 mol% H₂SO₄ bezogen auf DDP umgesetzt werden und in einem zweiten Schritt das DDP-OOH in Gegenwart der Cu-(II)-verbindung und des Verdünnungsmittels umgesetzt werden, wobei während der Reaktion ein Azeotrop enthaltend Wasser und Verdünnungsmittel abdestilliert wird, wobei
die Reaktion in Gegenwart von Additiven der Formel
HX-[A]-YH,
durchgeführt wird, wobei
X und Y unabhängig voneinander O oder N-R bedeuten, wobei R = H oder ein organischer Rest mit 1 bis 10 Kohlenstoffatomen ist,
und
A ein organischer Rest umfassend 2 bis 100 Kohlenstoffatome ist.

11. Verfahren nach Anspruch 10, **dadurch gekennzeichnet, dass** die Menge an H₂SO₄ 0,1 bis 3 mol% bezogen auf DDP beträgt.

12. Verfahren nach Anspruch 10 oder 11, **dadurch gekennzeichnet, dass** 0,8 bis 1,25 molare Equivalente, bezogen auf DDP, an Wasserstoffperoxid verwendet werden.

13. Verfahren nach mindestens einem der Ansprüche 10 bis 12, **dadurch gekennzeichnet, dass** nach beendeter Reaktion eine Filtration und Waschvorgänge angeschlossen werden.

14. Verfahren nach mindestens einem der Ansprüche 10 bis 13, **dadurch gekennzeichnet, dass** zumindest zum Teil Waschwässer vorheriger Ansätze verwendet werden.

15. Verfahren nach mindestens einem der Ansprüche 10 bis 14, **dadurch gekennzeichnet, dass** der Wassergehalt des DDP-OOH im Bereich von 5 bis 40 Gew.% liegt.

## Claims

1. Process for the preparation of 11(12)-pentadecen-15-olides starting from 1-hydroperoxy-16-oxabicyclo[10.4.0]hexadecane (DDP-OOH) in the presence of a Cu(II) compound and a diluent, **characterized in that** an azeotrope comprising water and the diluent is distilled off during the reaction, wherein
the reaction is carried out in the presence of additives of the formula
HX-[A]-YH
wherein
X and Y independently of one another denote O or N-R, wherein R = H or is an organic radical having 1 to 10 carbon atoms, and
A is an organic radical comprising 2 to 100 carbon atoms.

2. Process according to claim 1, **characterized in that** the amount of Cu (II) compound is 0.02 to 1.5 mol%, based on the DDP-OOH.

3. Process according to claim 1 or 2, **characterized in that** the weight ratio of DDP-OOH to the total amount of the diluent used in the cleavage is in the range of 1 : 2 to 1 : 7.

4. Process according to at least one of claims 1 to 3, **characterized in that** at least some of the diluent is recovered from previous batches.

5. Process according to at least one of claims 1 to 4, **characterized in that** the temperature of the reaction is in the range of from 80 to 100 °C.

6. Process according to at least one of claims 1 to 5, **characterized in that** the additives are polyalkylene glycols.

7. Process according to at least one of claims 1 to 6, **characterized in that** the amount of additives is 0.5 to 15 wt.%, based on the DDP-OOH.

8. Process according to at least one of claims 1 to 7, **characterized in that** the water content of the DDP-OOH is advantageously in the range of from 5 to 40 wt.%.

9. Process according to at least one of claims 1 to 8, **characterized in that** the process is carried out under pressures in the range of from 50 to 800 mbar.

10. Process for the preparation of 11(12)-pentadecen-15-olides according to claim 1, wherein in a first step 13-oxabicyclo[10.4.0]hexadec-1(12)-ene (DDP) and hydrogen peroxide are reacted in the presence of acetic acid and 0.05 to 5 mol% of H₂SO₄, based on the DDP, and in a second step the DDP-OOH is reacted in the presence of the Cu(II) compound and the diluent, wherein an azeotrope comprising water and the diluent is distilled off during the reaction, wherein
the reaction is carried out in the presence of additives of the formula
HX-[A]-YH
wherein
X and Y independently of one another denote O or N-R, wherein R = H or is an organic radical having 1 to 10 carbon atoms,
and
A is an organic radical comprising 2 to 100 carbon atoms.

11. Process according to claim 10, **characterized in that** the amount of H₂SO₄ is 0.1 to 3 mol%, based on the DDP.

12. Process according to claim 10 or 11, **characterized in that** 0.8 to 1.25 molar equivalents, based on the DDP, of hydrogen peroxide are used.

13. Process according to at least one of claims 10 to 12, **characterized in that** a filtration and washing operations follow after the reaction has ended.

14. Process according to at least one of claims 10 to 13, **characterized in that** at least some of the wash waters of previous batches is used.

15. Process according to at least one of claims 10 to 14, **characterized in that** the water content of the DDP-OOH is in the range of from 5 to 40 wt.%.

## Revendications

1. Procédé de préparation de 11(12)-pentadécén-15-olides à partir de 1-hydroperoxy-16-oxabicyclo[10.4.0]hexadécane (DDP-OOH) en présence d'un composé de cuivre (II) et d'un diluant, **caractérisé en ce que**, pendant la réaction, un azéotrope contenant de l'eau et du diluant est enlevé par distillation, la réaction se faisant en présence d'additifs de la formule
HX-[A]-YH,
dans laquelle
X et Y sont indépendamment l'un de l'autre O ou N-R, où R est H ou un résidu organique ayant de 1 à 10 atomes de carbone, et
A est un résidu organique comprenant de 2 à 100 atomes de carbone.

2. Procédé selon la revendication 1, **caractérisé en ce que** la quantité du composé de cuivre (II) est de 0,02 à 1,5% molaire, ramenée au DDP-OOH.

3. Procédé selon la revendication 1 ou la revendication 2, **caractérisé en ce que** le ratio en poids du DDP-OOH sur la quantité totale du diluant utilisé dans la dissociation se situe dans la plage de 1:2 à 1:7.

4. Procédé selon l'une au moins des revendications 1 à 3, **caractérisé en ce qu'**une partie au moins du diluant des mélanges précédents est récupérée.

5. Procédé selon l'une au moins des revendications 1 à 4, **caractérisé en ce que** la température réactionnelle se situe dans la plage de 80 à 100°C.

6. Procédé selon l'une au moins des revendications 1 à 5, **caractérisé en ce que** les additifs sont des polyalkylène glycols.

7. Procédé selon l'une au moins des revendications 1 à 6, **caractérisé en ce que** la quantité des additifs est de 0,5 à 15% en poids, ramenée au DDP-OOH.

8. Procédé selon l'une au moins des revendications 1 à 7, **caractérisé en ce que** la teneur en eau du DDP-OOH se situe avantageusement dans la plage de 5 à 40% en poids.

9. Procédé selon l'une au moins des revendications 1 à 8, **caractérisé en ce que** le procédé est mis en oeuvre à des pressions dans la plage de 50 à 800 mbars.

10. Procédé de préparation de 11(12)-pentadécén-15-olides selon la revendication 1, dans lequel, dans une première étape, du 13-oxabicyclo[10.4.0]hexadéc-1(12)-ène (DDP) et du peroxyde d'hydrogène sont mis à réagir en présence d'acide acétique et de 0,05 à 5% molaires de H₂SO₄, ramenés au DDP, dans une deuxième étape le DDP-OOH est mis à réagir en présence du composé de cuivre (II) et du diluant, et un azéotrope contenant de l'eau et du diluant est enlevé par distillation pendant la réaction, la réaction se faisant en présence d'additifs de la formule
HX-[A]-YH,
dans laquelle
X et Y sont indépendamment l'un de l'autre O ou N-R, où R est H ou un résidu organique ayant de 1 à 10 atomes de carbone, et
A est un résidu organique comprenant de 2 à 100 atomes de carbone.

11. Procédé selon la revendication 10, **caractérisé en ce que** la quantité de H₂SO₄ est de 0,1 à 3% molaires, ramenée au DDP.

12. Procédé selon la revendication 10 ou la revendication 11, **caractérisé en ce que** l'on utilise de 0,8 à 1,25 équivalent molaire de peroxyde d'hydrogène, ramenés au DDP.

13. Procédé selon l'une au moins des revendications 10 à 12, **caractérisé en ce que**, une fois la réaction terminée, on effectue une filtration et des opérations de lavage.

14. Procédé selon l'une au moins des revendications 10 à 13, **caractérisé en ce que**, du moins en partie, des eaux de lavage des mélanges précédents sont utilisées.

15. Procédé selon l'une au moins des revendications 10 à 14, **caractérisé en ce que** la teneur en eau du DDP-OOH se situe dans la plage de 5 à 40% en poids.
